(19) Europäisches Patentamt
European Patent Office
Office européen des brevets

(11) **EP 4 215 271 A1**

(12) **EUROPEAN PATENT APPLICATION**

(43) Date of publication:
**26.07.2023 Bulletin 2023/30**

(21) Application number: **22382034.1**

(22) Date of filing: **19.01.2022**

(51) International Patent Classification (IPC):
*B01J 27/18* (2006.01)      *C01B 25/32* (2006.01)
*C07C 29/00* (2006.01)      *C07C 31/02* (2006.01)
*C10L 1/02* (2006.01)

(52) Cooperative Patent Classification (CPC):
(C-Sets available)
**B01J 35/1061; B01J 27/18; B01J 35/0013;
B01J 35/023; B01J 35/10; B01J 35/1066;
B01J 35/1071; B01J 35/1076; C01B 25/327;
C07C 29/153** (Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **B. Braun Surgical, S.A.**
  **08191 Rubi (Barcelona) (ES)**
• **Universitat Politècnica de Catalunya**
  **08034 Barcelona (ES)**

(72) Inventors:
• **Turón Dols, Pau**
  **08191 Rubí (Barcelona) (ES)**
• **Alemán Llansó, Carlos Enrique**
  **08019 Barcelona (ES)**
• **Arnau, Marc**
  **08019 Barcelona (ES)**
• **Sans, Jordi**
  **08019 Barcelona (ES)**

(74) Representative: **Patentanwälte
Ruff, Wilhelm, Beier, Dauster & Partner mbB
Kronenstraße 30
70174 Stuttgart (DE)**

(54) **POROUS PERMANENTLY POLARIZED HYDROXYAPATITE, A PROCESS FOR ITS PRODUCTION AND USES THEREOF**

(57)    The invention relates to a porous permanently polarized hydroxyapatite having a mean pore diameter from 10 nm to 10.000 nm.

Further, the invention relates to a process for obtaining the porous permanently polarized hydroxyapatite, a composition or material comprising the porous permanently polarized hydroxyapatite, the use of the porous permanently polarized hydroxyapatite or the composition or material as a catalyst and a composition for preparing the porous permanently polarized hydroxyapatite.

**Fig. 1**

EP 4 215 271 A1

EP 4 215 271 A1

(52) Cooperative Patent Classification (CPC): (Cont.)

C-Sets
**C07C 29/153, C07C 31/08**

**Description**

FIELD OF THE INVENTION

The present invention relates to a porous permanently polarized hydroxyapatite, a process for its production, a composition or material comprising a porous permanently polarized hydroxyapatite, uses thereof and a composition for producing a porous permanently polarized hydroxyapatite.

BACKGROUND OF THE INVENTION

Mimicking the reactor morphologies found in nature has attracted scientists for their drastically enhanced catalytic activity. Indeed, confining the volume of chemical reactions to the micro- and nanoscales offers numerous advantages, such as the increase of the surface-to-volume ratio and the control on the heat and mass transfer, which are translated to an enhanced final selectivity and efficiency (Gaitzsch, J.; Huang, X.; Voit, B. Engineering Functional Polymer Capsules toward Smart Nanoreactors. Chem. Rev. 2016, 116, 1053-1093; Renggli, K.; Baumann, P.; Langowska, K.; Onaca, O.; Bruns, N.; Meier, W. Selective and Responsive Nanoreactors. Adv. Funct. Mater. 2011, 21, 1241-1259; Vriezema, D. M.; Aragonès, M. C.; Elemans, J. A. A. W.; Cornelissen, J. J. L. M.; Rowan, A. E.; Nolte, R. J. M. Self-Assembled Nanoreactors. Chem. Rev. 2005, 105, 1445-1489).

In recent studies it was reported the utilization of hydroxyapatite (HAp), $Ca_{10}(PO_4)_6(OH)_2$, for nitrogen and carbon fixation under mild reaction conditions (Revilla-López, G.; Sans, J.; Casanovas, J.; Bertran, O.; Puiggalí, J.; Turon, P.; Alemán, C. Analysis of Nitrogen Fixation by a Catalyst Capable of Transforming N2, CO2 and CH4 into Amino Acids under Mild Reactions Conditions. Appl. Catal. A Gen. 2020, 596, 117526; Sans, J.; Revilla-López, G.; Sanz, V.; Puiggalí, J.; Turon, P.; Alemán, C. Permanently Polarized Hydroxyapatite for Selective Electrothermal Catalytic Conversion of Carbon Dioxide into Ethanol. Chem. Commun. 2021, 57, 5163-5166) which postulates as a green and cheap alternative to conventional catalysts, showing an excellent plasticity behavior (Sans, J.; Sanz, V.; L. J. del Valle; Puiggalí, J.; Turon, P.; Aleman, C. Optimization of Permanently Polarized Hydroxyapatite Catalyst. Implications for the Electrophotosynthesis of Amino Acids by Nitrogen and Carbon Fixation. J. Catal. 2021, 397, 98-107).

Catalytic activation of HAp was developed by applying a thermally stimulated polarization (TSP) treatment to sintered pellets previously obtained by compressing HAp powder. Although the TSP treatment forces the permanent alignment of the $OH^-$ groups contained in the lattice through a specific direction and confers both electrical and electrochemical properties (Sans, J.; Arnau, M.; Turon, P.; Aleman, C. Article OH-. Regulating the Superficial Vacancies and OH- Orientations on Polarized Hydroxyapatite Electrocatalysts. Adv. Mater. Interfaces 2021, in press (DOI: 10.1002/admi.202100163); Rivas, M.; delValle, L. J.; Armelin, E.; Bertran, O.; Turon, P.; Puiggalí, J.; Alemán, C. Hydroxyapatite with Permanent Electrical Polarization: Preparation, Characterization, and Response against Inorganic Adsorbates. ChemPhysChem 2018, 19,1746-1755), the homogeneous and dense structure of catalytic HAp (hereafter denoted HAp/c) pellets restricts the efficiency of the catalyst and, therefore, the final yield of the reactions.

Thus, despite the progress already made, it remains further need for permanently polarized hydroxyapatite and respective compositions exhibiting a further optimized catalytic activity.

OBJECT AND SOLUTION

In view of the foregoing, the object underlying the present invention is therefore to make available a composition or material, a process for its production and uses thereof which properly address the above-mentioned need.

This object is accomplished by a porous permanently polarized hydroxyapatite according to claims 1 and 13, a process for producing the porous permanently polarized hydroxyapatite according to claim 6, a composition or material comprising a porous permanently polarized hydroxyapatite according to claim 14, use of the porous permanently polarized hydroxyapatite or composition or material comprising a porous permanently polarized hydroxyapatite according to claim 15 and a composition for preparing a porous permanently polarized hydroxyapatite according to claim 16. Preferred embodiments of the invention are defined in the dependent claims 2 to 5 and 7 to 12. Further preferred embodiments of the invention are defined in the present description. The subject-matter and wording, respectively of all claims is hereby incorporated into the description by explicit reference.

According to a first aspect, the present invention relates to a porous, in particular open-pored, permanently polarized hydroxyapatite, i.e. a permanently polarized hydroxyapatite comprising pores, in particular open pores.

Principally, the pores of the porous permanently polarized hydroxyapatite may have any shape or form. However, preferably, the pores are in the form of spherical pores.

Further, the pores of the porous permanently polarized hydroxyapatite may be in the form of interconnected pores, i.e. pores exhibiting interconnectivity.

Further, the pores of the porous permanently polarized hydroxyapatite may be present on or onto an outer surface of the porous permanently polarized hydroxyapatite, i.e. may be in the form of superficial pores, and/or may be present on or onto an inner surface or may be part of an inner surface of the porous permanently polarized hydroxyapatite, i.e. may be present inside the porous permanently polarized hydroxyapatite or may be in the form of inner pores. Preferably, the porous permanently polarized hydroxyapatite comprises both superficial pores and inner pores.

The porous permanently polarized hydroxyapatite comprises or has a mean pore diameter, in particular determined by means of Electron Microscopy (EM) or Electron Microscopy (EM) techniques, from 1 nm to 500.000 (in words: five hundred thousand) nm.

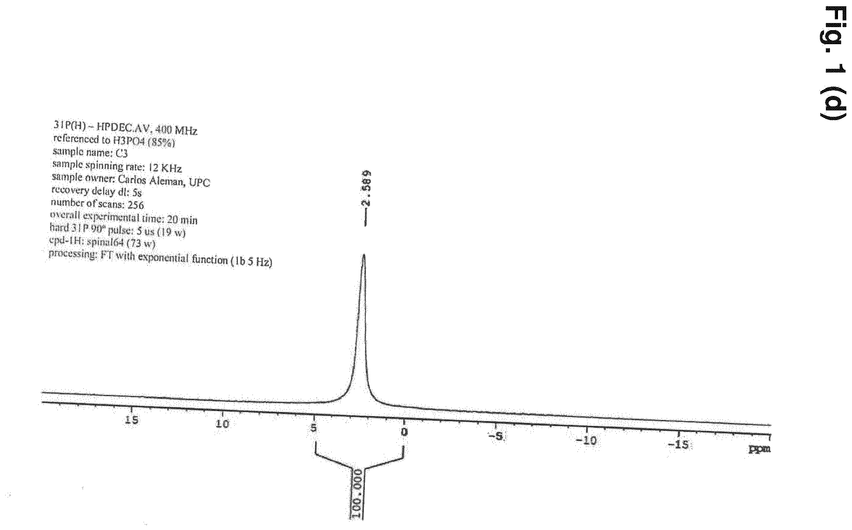

The term "porous permanently polarized hydroxyapatite" as used according to the present invention means a porous hydroxyapatite, in particular a synthetic porous hydroxyapatite, that has undergone a complete structural redistribution, in particular almost perfect, with a high crystallinity degree, i.e. particularly with a low amount of amorphous calcium phosphate and the presence of vacancies detected by increased electrochemical activity and the accumulation of charge per unit mass and surface. It has an electrochemical activity and ionic mobility which do not disappear over. The porous permanently polarized hydroxyapatite may have a $^{31}$P-NMR spectrum as shown on Fig. 1(d).

The present invention rests on the surprising finding that the catalytic activity, in particular heterogeneous catalytic activity, of permanently polarized hydroxyapatite is advantageously boostered by the presence of pores having a mean diameter as mentioned above. Such a porosity in turn advantageously results in a proper wettability of the permanently polarized hydroxyapatite which is especially advantageous in terms of an improved gas diffusion through active sites of the permanently polarized hydroxyapatite and the presence of water in a solid-liquid-gas interface. Thus, in particular heterogeneous catalytic fixation of carbon and/or nitrogen from molecular gases can be advantageously promoted. For example, the inventors were able to demonstrate that the carbon fixation from carbon dioxide ($CO_2$) and methane ($CH_4$) to yield ethanol improved by more than 3000 % when compared with conventional permanently polarized hydroxyapatite. Similarly, the inventors could prove that the synthesis of ammonia ($NH_3$) by dinitrogen ($N_2$) fixation increased by more than 2000 %. Therefore, the porous permanently polarized hydroxyapatite according to the present invention exhibits extraordinary potential for scalability and industrial utilization for many chemical reactions, in particular enabling a feasible green chemistry alternative to catalysts based on heavy metals.

In an embodiment of the invention, the mean pore diameter, in particular determined by means of Electron Microscopy (EM) or Electron Microscopy (EM) techniques, is from 10 nm to 10.000 (in words: ten thousand) nm, in particular 50 nm to 1.000 (in words: one thousand) nm, preferably 60 nm to 600 nm, more preferably 100 nm to 350 nm. The afore-mentioned pore diameters are especially useful for realizing the above-mentioned advantages of the present invention.

In a further embodiment of the invention, the porous permanently polarized hydroxyapatite has nanometric pores and/or submicrometric pores and/or micrometric pores. Preferably, the porous permanently polarized hydroxyapatite has nanometric pores and submicrometric pores or nanometric pores, submicrometric pores and micrometric pores. Thus, the selectivity of catalyzed reactions may be advantageously improved. Further, a change from a batch process to a continuous process may be facilitated, if desired, due to an improvement in the mass transfer.

The term "nanometric pores" as used according to the present invention means pores having a mean diameter, in particular determined by means of Electron Microscopy (EM) or Electron Microscopy (EM) techniques, from 1 nm to 100 nm, in particular 10 nm to 90 nm, preferably 50 nm to 80 nm.

The term "submicrometric pores" as used according to the present invention means pores having a mean diameter, in particular determined by means of Electron Microscopy (EM) or Electron Microscopy (EM) techniques, from > 100 nm to < 1000 nm, in particular 101 nm to 999 nm, in particular 120 nm to 900 nm, preferably 150 nm to 200 nm.

The term "micrometric pores" as used according to the present invention refers to pores having a mean diameter, in particular determined by means of Electron Microscopy (EM) or Electron Microscopy (EM) techniques, from 1 $\mu$m to 500 $\mu$m, in particular 1 $\mu$m to 100 $\mu$m or 2 $\mu$m to 100 $\mu$m, preferably 1 $\mu$m to 10 $\mu$m, more preferably 5 $\mu$m to 10 $\mu$m.

Advantageously, the additional presence of submicrometric pores and/or micrometric pores promotes the catalytic activity of the porous permanently polarized hydroxyapatite.

In a further embodiment of the invention, the nanometric pores have a mean pore diameter, in particular determined by means of Electron Microscopy (EM) or Electron Microscopy (EM) techniques, from 1 nm to 100 nm, in particular 10 nm to 90 nm, preferably 50 nm to 80 nm.

In a further embodiment of the invention, the submicrometric pores have a mean pore diameter, in particular determined by means of Electron Microscopy (EM) or Electron Microscopy (EM) techniques, from > 100 nm to < 1000 nm, in particular 101 nm to 999 nm, in particular 120 nm to 900 nm, preferably 150 nm to 200 nm.

In a further embodiment of the invention, the micrometric pores have a mean pore diameter, in particular determined by means of Electron Microscopy (EM) or Electron Microscopy (EM) techniques, from 1 $\mu$m to 500 $\mu$m, in particular 1 $\mu$m to 100 $\mu$m, preferably 1 $\mu$m to 10 $\mu$m.

Further, the porous permanently polarized hydroxyapatite may have a porosity, in particular determined by means of computing the percentage of void space observed by Scanning Electron Microscopy (SEM), from 0.1 % to 99 %, in particular 5 % to 50 %, preferably 10 % to 35 %.

Further, the porous permanently polarized hydroxyapatite may have a specific surface area (SSA), in particular determined by means of Brunauer-Emmett-Teller (BET) porosimetry, from 1 $m^2/g$ to 100 $m^2/g$, in particular 5 $m^2/g$ to 80 $m^2/g$, preferably 10 $m^2/g$ to 50 $m^2/g$.

Further, the porous permanently polarized hydroxyapatite may have a surface-area-to-volume ratio (sa/vol) from 30 $cm^{-1}$ to 150 $cm^{-1}$, in particular 40 $cm^{-1}$ to 120 $cm^{-1}$, preferably 60 $cm^{-1}$ to 85 $cm^{-1}$.

Further, the porous permanently polarized hydroxyapatite may have a crystallinity, in particular determined by means of wide angle x-ray scattering (WAXS), $\geq$ 65 %, in particular $\geq$ 70 %, preferably $\geq$ 75 %, more preferably from 75 % to 99.9 %.

Further, the porous permanently polarized hydroxyapatite may comprise crystallites having a mean size, preferably mean diameter, in particular determined by means of wide angle X-ray scattering (WAXS) or wide angle X-ray diffraction (WAXD), from 20 nm to 500 nm, in particular 50 nm to 200 nm, preferably 70 nm to 100 nm.

Further, the porous permanently polarized hydroxyapatite may have a water flow absorption, in particular determined by means of Optical Microscopy (in particular equipped with contact angle goniometer), from 0.1 $\mu$L/s to 20 $\mu$L/s, in particular 0.2 $\mu$L/s to 10 $\mu$L/s, preferably 1 $\mu$L/s to 6 $\mu$L/s.

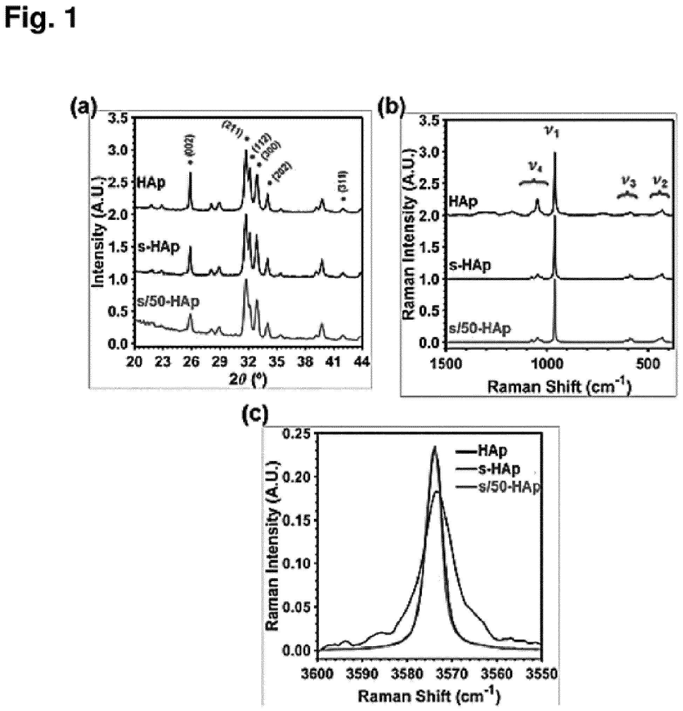

In a further embodiment of the invention, the porous permanently polarized hydroxyapatite has a wide angle X-ray scattering (WAXS) pattern or wide angle X-ray diffraction (WAXD) pattern as shown on Fig. 1(a). The wide angle X-ray scattering (WAXS) pattern or wide angle X-ray diffraction (WAXD) pattern shows peaks, in particular representative or unique peaks, at $2\theta$ = 25.9°, 31.7°, 32.1°, 32.8°, 34.0° and 39.8°. Preferably, said pattern is carried out or obtained at room temperature, preferably at a temperature of 20 °C to 25 °C, and/or under atmospheric conditions, in particular under atmospheric humidity and/or atmospheric pressure.

The term "room temperature" as used according to the present invention means a temperature from 15 °C to 35 °C, in particular 18 °C to 30 °C, preferably 20 °C to 30 °C, more preferably 20 °C to 28 °C, particularly 20 °C to 25 °C.

In a further embodiment of the invention, the porous permanently polarized hydroxyapatite has a Raman spectrum as shown on Fig. 1(b). The Raman spectrum shows peaks, in particular representative or unique peaks, at $v1$ = 962 $cm^{-1}$, $v2$ = 400 $cm^{-1}$ - 480 $cm^{-1}$, $v3$ = 570 $cm^{-1}$ - 625 $cm^{-1}$, and $v4$ = 1020 $cm^{-1}$ - 1095 $cm^{-1}$, and the $v$-OH at 3574 $cm^{-1}$ for hydroxyapatite and polarization peaks at 878 $cm^{-1}$, 848 $cm^{-1}$ and 794 $cm^{-1}$. Preferably, said spectrum is carried out or obtained at room temperature, preferably at a temperature of 20 °C to 25 °C, and/or under atmospheric conditions, in particular under atmospheric humidity and/or atmospheric pressure, in particular recorded using a laser with a wavelength at 532 nm.

In a further embodiment of the invention, the porous permanently polarized hydroxyapatite has a $^{31}$P-NMR spectrum as shown on Fig. 1(d). Said spectrum shows a unique peak at 2.6 ppm or around 2.6 ppm, i.e. in the range of 2.5 ppm to 2.7 ppm, corresponding to phosphate groups of hydroxyapatite. Preferably, said spectrum is carried out or obtained with solid porous permanently polarized hydroxyapatite at a temperature of 20 °C to 25 °C and using phosphoric acid ($H_3PO_4$) as reference. Further, said spectrum is preferably obtained or obtainable by using a 400 MHz or 400.1 MHz nuclear magnetic resonance (NMR) spectrometer. Generally, in particular depending on the used NMR spectrometer, the porous permanently polarized hydroxyapatite may have a $^{31}$P-NMR spectrum having a peak, in particular unique peak, in the range of 2.3 ppm to 2.9 ppm, in particular 2.4 ppm to 2.8 ppm, preferably 2.5 ppm to 2.7 ppm, more preferably 2.5 ppm to 2.6 ppm, corresponding to phosphate groups of the hydroxyapatite.

Further, the porous permanently polarized hydroxyapatite may have a proportion of amorphous calcium phosphate of > 0 % by weight to 17 % by weight, in particular from > 0 % by weight to 9 % by weight, preferably > 0 % by weight to 5 % by weight, based on the total weight of the porous permanently polarized hydroxyapatite.

Further, the porous permanently polarized hydroxyapatite may be free of amorphous calcium phosphate.

Further, the porous permanently polarized hydroxyapatite may have a proportion of tricalcium phosphate, in particular $\beta$-tricalcium phosphate, of > 0 % by weight to 35 % by weight, in particular > 0 % by weight to 12 % by weight, preferably > 0 % by weight to 5 % by weight, in particular < 0.5 % by weight, based on the total weight of the porous permanently polarized hydroxyapatite.

Further, the porous permanently polarized hydroxyapatite may be free of tricalcium phosphate, in particular $\beta$-tricalcium phosphate.

Further, the porous permanently polarized hydroxyapatite may have a bulk resistance of $10^7$ $\Omega$ $cm^2$ to $10^4$ $\Omega$ $cm^2$, in particular $10^7$ $\Omega$ $cm^2$ to $10^5$ $\Omega$ $cm^2$, preferably $10^5$ $\Omega$ $cm^2$. In particular, the bulk resistance may increase (only) from 0.1 % to 33 %, in particular 4 % to 63 %, preferably 4 %, after 3 months. The term "bulk resistance" as used according to the present invention means resistance to the electron transfer and may be determined by means of electrochemical impedance spectroscopy.

Further, the porous permanently polarized hydroxyapatite may have a surface capacitance which decreases less than 8 %, in particular less than 5 %, after 3 months. Preferably, the porous permanently polarized hydroxyapatite may have a surface capacitance which decreases from 0 % or > 0 % to 8 %, more preferably from 0 % or > 0 % to 5 %, after 3 months. The term "surface capacitance" as used according to the present invention means capacitance attributed to surface changes of hydroxyapatite induced by a thermal polarization process and may be determined by means of electrochemical impedance spectroscopy.

Preferably, the porous permanently polarized hydroxyapatite is free of a coating, i.e. free of any coating. Thus, blocking of pores due to the presence of a coating may be advantageously circumvented and the pores are able to exercise their promoting influence on the catalytic activity of the porous permanently polarized hydroxyapatite without any impairment.

Alternatively, the porous permanently polarized hydroxyapatite may have or comprise a coating.

Further, the porous permanently polarized hydroxyapatite may be in the form of particles. The particles may have a diameter, preferably mean diameter, in particular determined by means of Electron Microscopy (EM) or Electron Microscopy (EM) techniques, of 10 nm to 5.000 (in words: five thousand) nm, in particular 100 nm to 1.000 (in words: one thousand) nm, preferably 100 nm to 300 nm.

Further, the porous permanently polarized hydroxyapatite may be in the form of a powder, in particular having particles as mentioned in the preceding paragraph.

Further, the porous permanently polarized hydroxyapatite may be in the form of a shaped body. The shaped body may have a polygonal, for example triangular, quadratic or rectangular, pentagonal, hexagonal, heptagonal, octagonal or nonagonal, cross-section or a corner-less, in particular circular, oval-shaped or elliptical, cross-section.

In particular, the shaped body may be in the form of a disc, plate, cone (conus) or cylinder.

Further, the shaped body may have, for example, a thickness of > 0 cm to 10 cm, in particular > 0 cm to 1 cm, preferably > 0 cm to 0.2 cm.

Further, the porous permanently polarized hydroxyapatite is preferably in the form of a catalyst, in particular ceramic catalyst, preferably bioceramic catalyst.

According to a second aspect, the invention refers to a process for obtaining or preparing a porous permanently polarized hydroxyapatite, in particular according to the first aspect of the present invention. The process comprises the following steps:

(a) providing a mixture comprising or consisting of hydroxyapatite powder, in particular crystalline hydroxyapatite powder, and a pore-forming material,

(b) shaping or modelling the mixture provided in step (a) to obtain a shaped body comprising or consisting of the hydroxyapatite powder and the pore-forming material,

(c) sintering the shaped body obtained in step (b) to remove the pore-forming material and to obtain a shaped body comprising or consisting of porous hydroxyapatite,

(d) applying a constant or variable DC voltage, in particular between 250 V and 2550 V, or an equivalent electric field, in particular between 1.49 kV/cm and 15 kV/cm, or applying an electrostatic discharge, in particular between 2500 V and 1500000 V, or an equivalent electrical field, in particular between 148.9 kV/cm and 8928 kV/cm, to the shaped body obtained in step (c) to obtain a shaped body comprising or consisting of porous permanently polarized hydroxyapatite
and

(e) cooling the shaped body obtained in step (d) maintaining the DC voltage or the equivalent electric field applied in step (d)

or

cooling the shaped body obtained in step (d) maintaining the electrostatic discharge or the equivalent electric field applied in step (d)

or

cooling the shaped body obtained in step (d) without maintaining the DC voltage or the equivalent electric field applied in step (d)

or

cooling the shaped body obtained in step (d) without maintaining the electrostatic discharge or the equivalent electric field applied in step (d).

The term "pore-forming material" as used according to the present invention refers to a material or additive that is able to form or leave pores in, i.e. inside, and/or on, i.e. on the surface of, another material, in particular after its removal from said another material. The pore-forming material according to the present invention may also be termed as "pore-forming additive".

The term "hydroxyapatite powder" as used according to the present invention refers to a powder comprising or consisting of hydroxyapatite. The hydroxyapatite may be in the form of a natural, i.e. naturally occurring, hydroxyapatite and/or in the form of a synthetic hydroxyapatite. Further, the hydroxyapatite may be in the form of a crystalline hydroxyapatite.

Preferably, the mixture is provided in the form of a homogenous and/or pasty mixture, in particular in the form of an ink or ink composition, in step (a).

Further, the hydroxyapatite powder used in step (a) for providing the mixture may have a mean particle diameter, in particular determined by means of Electron Microscopy (EM) or Electron Microscopy (EM) techniques, from 10 nm to 5.000 (in words: five thousand) nm, in particular 50 nm to 1.000 (in words: one thousand) nm, preferably 100 nm to 1.000 (in words: one thousand) nm, more preferably 100 nm to 300 nm.

Further, the mixture provided in step (a) preferably has a proportion of the hydroxyapatite powder from 1 % by weight to 99.9 % by weight, in particular 10 % by weight to 90 % by weight, preferably 20 % by weight to 65 % by weight, based on the total weight of the mixture.

Further, the mixture provided in step (a) preferably has a proportion of the pore-forming material from 1 % by weight to 90 % by weight, in particular 10 % by weight to 80 % by weight, preferably 35 % by weight to 65 % by weight, based on the total weight of the mixture.

Advantageously, the pore diameter, in particular mean pore diameter, and the porosity of the porous permanently polarized hydroxyapatite may be controlled by the proportion of the pore-forming material used for providing the mixture in step (a).

Further, the mixture provided in step (a) additionally comprises a liquid, in particular water. The liquid, in particular water, may have a proportion from 10 % by weight to 90 % by weight, in particular 20 % by weight to 80 % by weight, preferably 40 % by weight to 60 % by weight, based on the total weight of the mixture.

Further, the step (a) may be carried out by adding first a part of the pore-forming material to the hydroxyapatite powder, in particular followed by stirring, and afterwards adding a remaining part of the pore-forming material to the hydroxyapatite powder, in particular followed by a further stirring.

Further, the step (a) may be carried out at a temperature from - 10 °C to 250 °C, in particular - 5 °C to 100 °C, preferably 0 °C to 25 °C. The temperature ranges disclosed in this paragraph are especially useful for providing a mixture exhibiting good rheological conditions which in turn is advantageous for carrying out step (b).

Further, between the step (a) and the step (b), the process may comprise a further step (ab) letting the mixture age, i.e. storing the mixture. Thus, a homogenous distribution of the pore-forming material in the mixture may be advantageously ensured. The step (ab) may be carried out at a temperature from - 10 °C to 250 °C, in particular - 5 °C to 100 °C, preferably 0 °C to 25 °C. Further, the step (ab) may be carried out for 1 min to 96 h, in particular 1 min to 24 h, preferably 1 min to 12 h, for example for 6 h.

Further, the hydroxyapatite powder used in step (a) may be prepared by using ammonium phosphate dibasic (diammonium hydrogen phosphate, $(NH_4)2HPO_4$) and calcium nitrate $(Ca(NO_3)_2)$ as reactants or starting materials. In particular, the hydroxyapatite powder may be prepared by carrying out the following steps:

$(a_{01})$ providing a mixture, in particular an aqueous mixture, preferably an aqueous-alcoholic mixture, of ammonium phosphate dibasic and calcium nitrate,

$(a_{02})$ stirring the mixture provided in step $(a_{01})$, in particular at room temperature,

$(a_{03})$ hydrothermal treating of the mixture stirred in step $(a_{02})$,

$(a_{04})$ cooling the mixture hydrothermally treated in step $(a_{03})$,

$(a_{05})$ separating precipitates obtained after cooling the mixture in step $(a_{04})$, and

$(a_{06})$ freeze-drying the precipitates separated in step $(a_{05})$ to produce hydroxyapatite, in particular crystalline hydroxyapatite.

The step $(a_{01})$ may be in particular carried out by using a mixture comprising or consisting of ammonium phosphate dibasic, calcium nitrate, water, in particular de-ionized water, ethanol, and optionally chelated calcium solutions. Advantageously, the pH value of the mixture and/or the pH value of an aqueous calcium nitrate solution applied for providing the mixture may be adjusted to 10-12, preferably 11. Thus, shapes and sizes of hydroxyapatite, in particular in the form of nanoparticles, can be controlled. Further, the step $(a_{02})$ may be carried out under agitation, in particular gentle agitation, for example applying 150 rpm to 400 rpm. Further, the step $(a_{02})$ may be carried out for 1 min to 12 h, in particular for 1 h. The step $(a_{02})$ may also be termed as an aging step, according to the present invention. Further, the step $(a_{03})$ may be carried out at a temperature of 60 °C to 240 °C, preferably of 150 °C. Further, the step $(a_{03})$ may be carried out at a pressure of 1 bar to 250 bar, preferably of 20 bar. Further, the step $(a_{03})$ may be carried out for 0.1 h to 72 h, preferably for 24 h. Further, the step $(a_{04})$ may be carried out by cooling the mixture hydrothermally treated in step $(a_{03})$ to a temperature of 0 °C to 90 °C, in particular of 25 °C. Further, the step $(a_{05})$ may be carried out by means of centrifugation and/or filtration. Further, the precipitates separated in step $(a_{05})$ may be washed, in particular using water and/or a mixture of ethanol and water, before the step $(a_{06})$ is carried out. Further, the step $(a_{06})$ may be carried out for 1 day to 4 days, in particular for 2 days to 3 days, preferably for 3 days.

Further, the step (b) is preferably carried out by means of extrusion, in particular robotic extrusion, i.e. robot-assisted extrusion.

More preferably, the step (b) is carried out by means of 3D printing or additive manufacturing, in particular robotic 3D printing or additive manufacturing, i.e. robot-assisted 3D printing or additive manufacturing.

Further, the step (b) may be carried out at a temperature from - 10 °C to 250 °C, in particular - 5 °C to 100 °C, preferably 0 °C to 25 °C.

Further, the step (c) is preferably carried out at a temperature between 700 °C and 1200 °C, in particular 700 °C and 1150 °C, preferably 800 °C and 1100 °C, in particular at 1000 °C.

Further, the process preferably comprises between the step (c) and the step (d) a further step (cd)

-   pressing the shaped body obtained in step (c) to obtain a pressed shaped body.

In particular, the step (cd) may be carried out under a pressure of 1 MPa to 1000 MPa, in particular 100 MPa to 800 MPa, preferably 600 MPa to 700 MPa. Further, the step (cd) may be carried out for 1 min to 90 min, in particular 5 min to 50 min, preferably 10 min to 30 min.

The pressed shaped body may have a polygonal, for example triangular, quadratic or rectangular, pentagonal, hexagonal, heptagonal, octagonal or nonagonal, or a corner-less, in particular circular, oval-shaped or elliptical, cross-section. Further, the pressed shaped body may have a thickness of > 0 cm to 10 cm, in particular > 0 cm to 5 cm, preferably > 0 cm to 2 cm. In particular, the pressed shaped body may have a thickness of 0.1 cm to 10 cm, in particular 0.1 cm to 5 cm, preferably 0.5 cm to 2 cm.

Preferably, the pressed shaped body are in the form of a disc, plate, cone or cylinder.

Advantageously, by carrying out the step (d), catalytic activation of the shaped body or the porous hydroxyapatite obtained in step (c) may be accomplished. Preferably, the step (d) is carried out by placing the shaped body obtained in step (c) between a positive electrode and a negative electrode, preferably wherein the shaped body obtained in step (c) is in contact with both electrodes, i.e. the positive electrode and the negative electrode. The electrodes may, by way of example, be in the form of stainless steel plates, in particular stainless steel AISI 304 plates. Further, the electrodes may have a mutual distance of 0.01 mm to 10 cm, in particular 0.01 mm to 5 cm, preferably 0.01 mm to 1 mm.

Further, the electrodes may be of different shapes. The electrodes may have a polygonal cross-section, for example quadratic or rectangular, or a corner-less, in particular circular, oval-shaped or elliptical, cross-section. In particular, the electrodes may have a thickness of > 0 cm to 10 cm, in particular > 0 cm to 5 cm, preferably > 0 cm to 1 mm. For example, the electrodes may be in the form of a disc, plate or cylinder.

Further, the constant or variable DC voltage or the equivalent electric field may be applied in the aforementioned step (d) for 1 h to 24 h, in particular 0.1 h to 10 h, in particular 1 h.

Further, the DC voltage applied in the aforementioned step (d) is preferably 500 V, which is equivalent to a constant electric field of 3 kV/cm.

Further, the equivalent electric field applied in the aforementioned step (d) is preferably 3 kV/cm.

Further, the step (d) is preferably carried out at a temperature of at least 900 °C, more preferably at least 1000 °C. Preferably, the step (d) is carried out at a temperature of 900 °C to 1200 °C, in particular 1000 °C to 1200 °C, particularly 1000 °C.

Further, the step (d) may be preferably carried out for > 0 min to 24 h or for at least 1 minute.

Preferably, the step (d) is carried out by applying a constant or variable DC voltage of 500 V at 1000 °C for 1 h to the shaped body obtained in step (c).

Further, the aforementioned step (e) may be carried out by cooling the shaped body obtained in step (d) to room temperature.

Further, the aforementioned step (e) may be carried out for 1 min to 72 h, in particular 15 min to 5 h, preferably 15 min to 2 h.

In a further embodiment of the invention, the pore-forming material comprises or is a polymer, in particular a synthetic polymer, i.e. a polymer produced in a laboratory, or a biopolymer, i.e. a naturally occurring polymer.

In a further embodiment of the invention, the polymer is selected from the group consisting of poloxamer, polyolefines, polyesters, polyamides, polyimides, polyvinyl alcohols, polyurethanes, polycarbonates, polyalkylterephthalates, polyarylterephthalates, polyaryletherketones, polyhydroxyalkanoates, proteins such as extracellular proteins and/or globular proteins and/or enzymes and/or antibodies and/or blood clotting factors, polysaccharides and mixtures of at least two of the afore-said polymers.

In a further embodiment of the invention, the polymer is a poloxamer. Poloxamers are nonionic triblock copolymers comprising or consisting of a central hydrophobic chain of polyoxypropylene (poly(propylene oxide)) flanked by two hydrophilic chains of polyoxyethylene (poly(ethylene oxide)). Poloxamers are also known by the tradenames Pluronic, Kolliphor (pharma grade) and Synperonic. For the generic term poloxamer, these copolymers are commonly named with the letter P (for poloxamer) followed by three digits: the first two digits multiplied by 100 give the molecular mass or approximate molecular mass of the polyoxypropylene core, i.e. the central hydrophobic chain of polyoxypropylene, and the last digit multiplied by 10 gives the percentage polyoxyethylene content (e.g. P407 = poloxamer with polyoxypropylene molecular mass of 4000 g/mol and a 70% polyoxyethylene content). For the Pluronic and Synperonic tradenames, coding of these copolymers starts with a letter to define its physical form at room temperature (L = liquid, P = paste, F = flake (solid)) followed by two or three digits. The first digit (two digits in a three-digit number) in the numerical designation, multiplied by 300, indicates the approximate molecular weight of the polyoxypropylene core and the last digit multiplied by 10 gives the percentage polyoxyethylene content (e.g. L61 indicates a polyoxypropylene molecular mass of 1,800 g/mol and a 10% polyoxyethylene content). In the example given, poloxamer 181 (P181) is equal to Pluronic L61 and Synperonic PE/L61.

Surprisingly, it turned out that especially poloxamer is able 1) to control precisely the final architecture and porosity of the porous permanently polarized hydroxyapatite without affecting its crystal structure; and 2) to be eliminated by the sintering step (c) without affecting the dimensional and mechanical stability of the final porous permanently polarized hydroxyapatite.

In a further embodiment of the invention, the poloxamer comprises a polyoxypropylene core, i.e. a central hydrophobic chain of polyoxypropylene or a central polyoxypropylene unit, having a molecular mass from 1 g/mol to 500.000 (in words: five hundred thousand) g/mol, in particular 1.000 (in words: one thousand) g/mol to 30.000 (in words: thirty thousand) g/mol, preferably 10.000 (in words: ten thousand) g/mol to 20.000 (in words: twenty thousand) g/mol.

Preferably, the poloxamer has a polyoxypropylene content, i.e. a proportion of polyoxypropylene, from 1 % by weight to 90 % by weight, in particular 10 % by weight to 80 % by weight, preferably 35 % by weight to 65 % by weight.

In a further embodiment of the invention, the poloxamer has a polyoxyethylene content, i.e. a proportion of polyoxyethylene, from 1 % by weight to 90 % by weight, in particular 10 % by weight to 80 % by weight, preferably 35 % by weight to 65 % by weight.

In a further embodiment of the invention, the polymer has a proportion from 1 % by weight to 90 % by weight, in particular 10 % by weight to 80 % by weight, preferably 20 % by weight to 80 % by weight, more preferably 35 % by weight to 65 % by weight, based on the total weight of the pore-forming material. For example, the polymer may have a proportion of 50 % by weight, 60 % by weight, 73 % by weight or 78 % by weight, based on the total weight of the pore-forming material.

Particularly, the polymer may have a proportion of ≤ 50 % by weight, in particular 20 % by weight to 50 % by weight, preferably 35 % by weight to 50 % by weight, based on the total weight of the pore-forming material. Surprisingly, it turned out that a proportion of the polymer as disclosed in this paragraph results in the formation of nanometric pores and submicrometric pores, in particular in the formation only of nanometric pores and submicrometric pores, preferably in the formation only of nanometric pores. Thus, by tuning the proportion of the polymer, the formation of submicrometric pores and/or micrometric pores may be avoided.

Alternatively, the polymer may particularly have a proportion of ≥ 50 % by weight, in particular > 50 % by weight, preferably 60 % by weight to 90 % by weight, more preferably 60 % by weight to 80 % by weight, based on the total weight of the pore-forming material. Surprisingly, it turned out that a proportion of the polymer as disclosed in this paragraph results not only in the formation of nanometric pores and submicrometric pores, but also in the formation of micrometric pores. Thus, the catalytic area may be advantageously increased. Further, the additional formation of submicrometric pores and micrometric pores may facilitate a continuous process by improving the mass transfer.

Further, the polymer proportions as disclosed in the preceding paragraphs have the (additional) advantage that the shaped body obtained in step (c) maintains the desired shape, in particular without showing observable cracks or shrinkage, and with good structural stability.

Further, the pore-forming material may preferably comprise a liquid, in particular water. More specifically, the liquid, in particular water, may preferably have a proportion from 10 % by weight to 90 % by weight, in particular 20 % by weight to 80 % by weight, preferably 40 % by weight to 60 % by weight, based on the total weight of the pore-forming material.

In a further embodiment of the invention, the pore-forming material is in the form of a gel, preferably hydrogel, i.e. water-containing gel.

According to a third aspect, the present invention refers to a porous permanently polarized hydroxyapatite obtained or obtainable by a process according to the second aspect of the present invention.

With respect to further features and advantages of the porous permanently polarized hydroxyapatite and the process for obtaining a porous permanently polarized hydroxyapatite, reference is made in its entirety to the previous description, i.e. the disclosure provided under the first aspect and second aspect of the present invention. The features and advantages described in the previous description in terms of the porous permanently polarized hydroxyapatite and the process for obtaining a porous permanently polarized hydroxyapatite, do also apply, mutatis mutandis, with respect to the porous permanently polarized hydroxyapatite according to the third aspect of the present invention.

According to a fourth aspect, the present invention refers to a composition or material comprising or consisting of a porous permanently polarized hydroxyapatite according to the first aspect or third aspect of the present invention.

Preferably, the composition or material is in the form of a catalyst, in particular ceramic catalyst, preferably bioceramic catalyst.

Further, the porous permanently polarized hydroxyapatite may have a proportion from 1 % by weight to 99.9 % by weight, in particular 10 % by weight to 90 % by weight, preferably 20 % by weight to 65 % by weight, based on the total weight of the composition or material.

The composition or material may further comprise an active ingredient. The active ingredient may be in particular a biologically or pharmaceutically active ingredient.The active ingredient may be in particular selected from the group consisting of antimicrobial, more particularly antibiotic, ingredient, wound healing-promoting ingredient, disinfecting ingredient, anti-inflammatory ingredient, blood coagulation-promoting ingredient, growth factors, cell-differentiating factors, cell-adhesive factors, cell-recruiting factors, cell receptors, cell-binding factors, cytokines, peptides, structural proteins, extracellular proteins such as, for example, collagen, serum proteins such as, for example, albumin, polysaccharides such as, for example, hyaluronic acid, oligonucleotides, polynucleotides, DNA, RNA, salts thereof, stereoisomers, more particularly diastereomers, thereof and mixtures thereof.

For example, the active ingredient may be selected from the group consisting of biguanides, polyhexamethylene biguanide (PHMB), triclosan, chlorhexidine, gentamicin, vitamins, copper, zinc, silver, gold and mixtures thereof.

The composition or material may further comprise a material being selected from the group consisting of polymer, ceramic, silicate, organo-metallic compounds and mixtures thereof.

The polymer may be a biodegradable polymer, i.e. a polymer which degrades in vivo, i.e. within a human or animal body, or a non-biodegradable polymer. Further, the polymer may be a biopolymer, i.e. a naturally occurring polymer, or a synthetic, i.e. technical or not naturally occurring polymer. Further, the polymer may be a homopolymer or a copolymer, i.e. a polymer comprising at least two, in particular only two or more, different monomeric units.

The polymer may be in particular selected from the group consisting of polyolefines, polyesters, polyamides, polyimides, polyvinyl alcohols, polyurethanes, polycarbonates, polyalkylterephthalates, polyarylterephthalates, polyaryletherketones, polyhydroxyalkanoates, proteins such as extracellular proteins and/or globular proteins and/or enzymes and/or antibodies and/or blood clotting factors, polysaccharides and mixtures thereof.

In particular, the polymer may be selected from the group consisting of polyethylene, low-density polyethylene, high-density polyethylene, high-molecular-weight polyethylene, ultra-high-molecular-weight polyethylene, polypropylene, polyethylene terephthalate, polypropylene terephthalate, polybutylene terephthalate, polyamide 6, polyamide 6-6, polyamide 6-12, poylamide 12, rayon, silk, in particular spider silk, polytetrafluorethylene, polyvinylidene dichloride, polyvinylidene difluoride, polytetrafluorpropylene, polyhexafluorpropylene, polyvinyl alcohol, polyglycolide, polylactide, polydioxanone, polyhydroxybutyrate, poly-3-hydroxybutyrate, poly-4-hydroxybutyrate, polytrimethylene carbonate, poly-$\epsilon$-caprolactone, collagen, gelatine, elastin, reticulin, fibronectin, laminin, fibrin, fibrinogen, albumin, starch, amylose, amylopectin, dextran, dextrin, cellulose, cellulose derivatives such as alkylcellulose, methylcellulose, hydroxyalkylcellulose, hydroxymethylcellulose, hydroxyethylcellulose, hydroxypropylcellulose, carboxyalkylcellulose, carboxymethylcellulose, chitin, chitosan, hyaluronic acid, dextran sulfate, heparin, heparan sulfate, chondroitin sulfate, dermatan sulfate, DNA, RNA, salts thereof, stereoisomers thereof, copolymers thereof and mixtures thereof.

The composition or material may further comprise an inorganic catalyst, in particular an inorganic photocatalyst. More specifically, the composition or material may be least partially, in particular only partially or completely, coated with an inorganic catalyst, in particular photocatalyst. The inorganic catalyst may be a catalyst such as $TiO_2$, $MgO_2$, $MnO_2$ or combinations thereof.

Further, the composition or material is preferably free of a coating, i.e. the composition or material is preferably free of any coating.

Alternatively, the composition or material may have or comprise a coating. In particular, the composition or material may be least partially, in particular only partially or completely, coated with aminotris(methylenephosphonic acid) and/or zirconium oxychloride ($ZrOCl_2$) and/or zirconia ($ZrO_2$). More specifically, the composition or material may have a three-layered coating, in particular wherein the three-layered coating may be composed of two layers of aminotris(methylenephosphonic acid) and a layer of zirconium oxychloride ($ZrOCl_2$) or zirconia ($ZrO_2$), preferably wherein the

layer of zirconium oxychloride is arranged or sandwiched between the two layers of aminotris(methylenephosphonic acid).

Further, the composition or material may be preferably a medical, in particular pharmaceutical, composition or material.

Further, the composition or material may be preferably a medical device, in particular an implant such as a bone implant or prosthesis, in particular knee or hip prosthesis.

With respect to further features and advantages of the composition or material, reference is made in its entirety to the previous description, i.e. the disclosure provided under the first aspect, second aspect and third aspect of the present invention. The features and advantages described in the previous description, in particular in terms of the porous permanently polarized hydroxyapatite, do also apply, mutatis mutandis, with respect to the composition or material according to the fourth aspect of the present invention.

According to a fifth aspect, the present invention refers to the use of a porous permanently polarized hydroxyapatite according to the first aspect or the third aspect of the present invention or to the use of a composition or material according to the fourth aspect of the present invention as a catalyst, in particular in a reaction for the synthesis or preparation of organic molecules, preferably ethanol, or in a reaction for the synthesis or preparation of inorganic molecules, preferably ammonia.

Especially preferably, the porous permanently polarized hydroxyapatite according to the first aspect or the third aspect of the present invention or the composition or material according to the fourth aspect of the present invention is used as a catalyst for the synthesis or preparation of ethanol or ammonia.

With respect to further features and advantages of the use, reference is made in its entirety to the previous description, i.e. the disclosure provided under the first aspect, second aspect, third aspect and fourth aspect of the present invention. The features and advantages described in the previous description in terms of the porous permanently polarized hydroxyapatite do also apply, mutatis mutandis, with respect to the use according to the fifth aspect of the present invention.

According to a sixth aspect, the present invention refers to a composition, in particular a pasty or ink composition, preferably 3D printing ink, in particular for obtaining or preparing a porous permanently polarized hydroxyapatite, in particular according to the first aspect or third aspect of the present invention. The composition comprises or consists of hydroxyapatite powder and a pore-forming material.

Preferably, the hydroxyapatite powder has a mean particle diameter, in particular determined by means of Electron Microscopy (EM) or Electron Microscopy (EM) techniques, from 10 nm to 5.000 (in words: five thousand) nm, in particular 50 nm to 1.000 (in words: one thousand) nm, preferably 100 nm to 1.000 (in words: one thousand) nm, more preferably 100 nm to 300 nm.

Further, the hydroxyapatite powder preferably has a proportion from 1 % by weight to 99.9 % by weight, in particular 10 % by weight to 90 % by weight, preferably 20 % by weight to 65 % by weight, based on the total weight of the composition.

Further, the pore-forming material preferably has a proportion from 1 % by weight to 90 % by weight, in particular 10 % by weight to 80 % by weight, preferably 35 % by weight to 65 % by weight, based on the total weight of the composition.

Further, the composition preferably comprises a liquid, in particular water. The liquid, in particular water, may in particular have a proportion from 10 % by weight to 90 % by weight, in particular 20 % by weight to 80 % by weight, preferably 40 % by weight to 60 % by weight, based on the total weight of the composition.

With respect to further features and advantages of the composition, reference is made in its entirety to the previous description, i.e. the disclosure provided under the first aspect, second aspect, third aspect, fourth and fifth aspect of the present invention. The features and advantages described in the previous description, in particular in terms of the porous permanently polarized hydroxyapatite and the pore-forming material, do also apply, mutatis mutandis, with respect to the composition according to the sixth aspect of the present invention.

Further features and advantages of the invention will become clear from the following figures, descriptions thereof and examples in conjunction with the subject-matter of the dependent claims. The individual features can be realized either singularly or severally in combination in one embodiment of the invention. The preferred embodiments merely serve for illustration and better understanding of the invention and are not to be understood as in any way limiting the invention.

BRIEF DESCRIPTION OF THE FIGURES

In the figures, the following is schematically displayed:

**Figure 1.** Structural characterization of as prepared HAp, s-HAp and s/50-HAp samples: (a) WAXD spectra, (b) Raman spectra in the region of the four characteristic vibrational modes ($v_1$- $v_4$), and (c) Stacked Raman spectra of the samples in the region of 3550 - 3600 cm$^{-1}$ corresponding to the v(O-H) vibrational mode.

**Figure 2** SEM micrographs of s-HAp and s/x-HAp samples obtained using different pluronic hydrogel mass percentages. Porosity was clearly observed when the hydrogel was introduced into the blend.

**Figure 3.** Dependence of (a) the pore size and (b) the porosity of s/x-HAp samples on the pluronic hydrogel wt%.

**Figure 4.** (a) HAp ink loaded with a 60 wt.-% of pluronic hydrogel extruded with a syringe. (b) Effect of sintering on the structure of the same HAp ink, as modeled with different shapes. (c) SEM inspection of porosity the outer face (c1) and the interior (c2) of a HAp ink.

**Figure 5.** Geometrical comparison of (a) 60-HAp/c cube, and (b) HAp/c disks.

**Figure 6.** (a) Comparison of the ethanol yields obtained from the $CO_2$ and $CH_4$ fixation reaction as catalyzed by HAp/c and 60-HAp/c. Reactions were performed using an initial $CO_2$ : $CH_4$ mixture (3 bar each), 1 mL of water, at 140 °C without UV irradiation for 48h. (b) Variation of the ethanol production as a function of the initial water content (expressed in $mmol/g_{cat}$ instead of $\mu mol/g_{cat}$). The rest of the reaction conditions were identical to those described for (a).

**Figure 7.** Comparison of the ammonium yields obtained from the $N_2$ fixation reaction as catalyzed by HAp/c and 60-HAp/c. Reactions were performed using an initial $N_2$ pressure (6 bar), 20 mL of water, at 120 °C with UV irradiation for 24 h.

**Figure 8.** Pore size distribution histograms for s/x-HAp samples.

EXPERIMENTAL SECTION

1. Materials

Calcium nitrate $Ca(NO_3)_2$, diammomium hydrogen phosphate [$(NH_4)_2HPO_4$; purity > 99.0%], ammonium hydroxide solution 30% [$NH_4OH$; purity: 28-30% w/w], zirconyl chloride ($ZrOCl_2·8H_2O$; ZC), aminotris(methylenephosphonic acid) (ATMP) and the initial Pluronic® F-127 polymer ($C_3H_6O·C_2H_4O)_x$, BioReagent powder) were purchased from Sigma Aldrich. Ethanol (purity > 99.5%) was purchased from Scharlab. $N_2$, $CH_4$ and $CO_2$ gases with a purity of > 99.995% were purchased from Messer. All experiments were performed with milli-Q water.

2. Synthesis of hydroxyapatite (HAp)

15 mL of 0.5 M $(NH_4)_2HPO_4$ in de-ionized water were added at a rate of 2 mL/min to 25 mL of a 0.5 M of $Ca(NO_3)_2$ solution in ethanol with pH previously adjusted to 11 using ammonium hydroxide solution. The mixture was left aging for 1 h under gentle agitation (150 rpm) at room temperature. Hydrothermal treatment at 150 °C was applied using an autoclave Digestec DAB-2 for 24 h. The autoclave was allowed to cool down before opening. The precipitates were separated by centrifugation and washed with water and a 60/40 v/v mixture of ethanol/water (twice). After freeze-drying it for 3 days, a white powder was obtained.

3. Synthesis of pluronic hydrogel

25 g of distilled water was mixed with 25 g of Pluronic® F-127 polymer using a FlackTek SpeedMixer at 3500 rpm for 5 minutes. After that, 50 g of Pluronic® polymer were added and vigorously stirred using the same conditions. The resultant hydrogel was stored at 4 °C.

4. Synthesis of porous HAp inks

HAp powder, hereafter denoted as prepared HAp, was obtained from a hydrothermal route and freeze-dried for 72 h to eliminate the water content. Pluronic hydrogel was prepared from a water and Pluronic® F-127 mixture.
HAp inks with desired weight percentage of pluronic hydrogel were obtained from the slowly addition of half of the weighted pluronic hydrogel to HAp powder, followed by rigorous stirring at 2500 rpm for 2 minutes using a Fisherbrand™ Digital Vortex Mixer. This process was repeated again adding the rest of hydrogel to achieve the homogeneous mixture. All the procedure was carried out at low temperature (i.e. in a cold room < 4 °C). The white paste obtained was left aging at 4 °C for 24 h to ensure that the pluronic hydrogel became homogeneously distributed. Finally, HAp inks were modeled at low temperatures to obtain the desired 3D HAp scaffolds and sintered at 1000 °C using a muffle Carbolite ELF11/6B/301 for 2 h. Hereafter, this product is denoted as s/x-HAp, where s refers to sintered and x corresponds to the mass percentage (%) of pluronic hydrogel. For terms of completion, sintered grains based on as prepared HAp powder were also prepared using the same temperature and time conditions (s-HAp).

5. Catalytic activation

s-HAp disks, which were obtained by pressing 150 mg of s-HAp powder at 620 MPa for 10 min, and s/x-HAp cubes were catalytically activated by placing the samples between two stainless steel plates (AISI 304) and applying a constant DC voltage of 500 V for 1 h with a GAMMA powder supply, while temperature was kept at 1000 °C. Samples were allowed to cool down maintaining the applied electric potential for 30 minutes, and finally, all the system was powered off and left to cool overnight. Hereafter, catalysts derived from s-HAp and s/x-HAp have been denoted HAp/c and x-HAp/c.

6. Characterization

Structural characterization was performed using wide angle X-ray diffraction (WAXD), Raman microscopy, scanning electron microscopy (SEM) and energy dispersive X-ray (EDX) analyses. Water absorption capabilities were obtained by means of a contact angle measuring equipment.

More specifically, structural analyses were performed by means of a inVia Qontor confocal Raman microscope (Renishaw) equipped with a Renishaw Centrus 2957T2 detector and a 532 nm laser. In order to obtain representative data, 32 single point spectra were averaged.

Wide angle X-ray scattering (WAXS) studies were conducted using a Brucker D8 Advance model with Bragg-Brentano $2\theta$ configuration and Cu $K_\alpha$ radiation ($\lambda$ = 0.1542 nm). Measurements were performed in a $2\theta$ range of 20°-60° in steps of 0.02° and scan speed of 2 s, using a onedimensional Lynx Eye detector. The crystallinity ($\chi_c$) was obtained using the following expression:

$$\chi_c = 1 - \frac{V_{112/300}}{I_{300}} \qquad \text{(S1)}$$

where $I_{300}$ is the intensity of the (300) reflection and $V_{112/300}$ is the intensity of the hollow between the (112) and (300) reflections, which disappears in non-crystalline samples. The crystallite size, $L_{hkl}$, was calculated using the Debye-Scherrer equation

$$L_{hkl} = \frac{0.9 \cdot \lambda}{B \cdot \cos\theta_{hkl}} \qquad \text{(S2)}$$

where $\lambda$ is the wavelength of the monochromatic X-ray beam, $B$ is the full width at half maximum of the peak at the maximum intensity, and $\theta_{hkl}$ is the peak diffraction angle that satisfies the Bragg's law for the (hkl) plane.

SEM studies were carried out using a Focused Ion Beam Zeiss Neon40 microscope operating at 5 kV equipped with an EDX (20 kV) spectroscopy system. The latter technique was used to estimate the composition of the distribution of the ATMP/ZC/ATMP coatings.

Water absorption capability was computed by means of a contact angle measuring equipment OCA 15EC (Data-Physics Instruments). To dispense the water droplet, a 500 $\mu$L DS500/GT glass syringe and a needle SNS 021/011 were used. The absorption flow rates were calculated by dropping 1.5 $\mu$L water droplets onto the surface of the samples while recording with a 30 fps camera. Then, the photograms were analyzed.

7. Structural Characterization

In order to achieve optimal catalytic activation, as prepared HAp powder was calcined at 1000 °C, enhancing the crystallinity, surface charge accumulation and OH⁻ vacancies through a dehydration process. Moreover, exposure of HAp inks at such high temperature is also necessary to confer mechanical stability and to eliminate the pluronic hydrogel (which could interfere in the catalyst performance, masking the catalytic activity). In this section, the structure and crystallinity of s-HAp and s/x-HAp are compared with the original as prepared HAp.

WAXD spectra of as prepared HAp, s-HAp and s/50-HAp samples are displayed in Figure 1a. All samples presented the characteristic peaks of HAp at $2\theta$ = 25.9°, 31.7°, 32.1°, 32.8°, 34.0° and 39.8°, which correspond to the (002), (211), (112), (300), (202) and (310) reflections, respectively (JCPDS card number 9-0077). Representative crystallographic parameters (see below table 1) were obtained to determine whether the suppression of pluronic hydrogel affected the dehydration process.

**Table 1.** Representative crystallographic parameters obtained for as prepared HAp, s-HAp and s/50-HAp samples.

|  | Hap | s-HAp | s/50-Hap |
|---|---|---|---|
| $X_c$ | 0.78 $\pm$ 0.01 | 0.81 $\pm$ 0.02 | 0.84 $\pm$ 0.02 |
| $L_{221}$ [nm] | 24 $\pm$ 2 | 24 $\pm$ 1 | 20 $\pm$ 1 |
| $L_{002}$ [nm] | 54 $\pm$ 3 | 39 $\pm$ 2 | 24 $\pm$ 2 |
| $I_{002}$ | 0.585 $\pm$ 0.02 | 0.438 $\pm$ 0.02 | 0.238 $\pm$ 0.02 |
| $I_{112}$ | 0.728 $\pm$ 0.04 | 0.717 $\pm$ 0.03 | 0.598 $\pm$ 0.05 |

The expected crystal refinement was observed for the two samples treated at high temperatures, slightly increasing their crystallinity ($\chi_c$; Eq S1) from 0.78 $\pm$ 0.01 for HAp to 0.84 $\pm$ 0.02 for s/50-HAp and 0.81 $\pm$ 0.02 for s-HAp. Nevertheless, some structural differences arose when the crystallite size of the (211) main reflection ($L_{211}$) was analyzed (Eq S2). A reduction of ~4 nm was observed for the s/50-HAp, suggesting that the presence of the hydrogel restricted the crystal growth during the crystal refinement process. Accordingly, this observation together with the fact that the crystallinity was higher for s/50-HAp than for s-HAp, indicates porosity promoting the formation of crystallization nuclei and, therefore, crystals are more abundant but slightly smaller for the former than for the latter. On the other hand, the intensities of the (002) and (112) reflections normalized by the intensity of the (211) main reflection ($I_{002}$ and $I_{112}$, respectively) were considerably lower for s/50-HAp, supporting such hypothesis. $I_{002}$ is commonly used to study the growth anisotropy through the c-axis of the HAp crystal lattice, also affected by sintering of HAp grains ($I_{002}{}^{HAp} > I_{002}{}^{s-HAp} > I_{002}{}^{s/50-HAp}$). These results allow to conclude that the pluronic hydrogel acts as a template, directing the shape and size of HAp grains that results in the generation of porosity.

Generation of other calcium phosphate phases, such as $\beta$-tricalcium phosphate ($\beta$-TCP), can be easily produced during the dehydration process. Although the characteristic reflection (021) at $2\theta$ = 31.5° of $\beta$-TCP (JCPDS card number 9-0432) was not observed in the WAXD spectra, the formation of other salts was not completely discarded as most of their reflections share peak positions with HAp. In addition, Raman microscopy measurements were performed in order to obtain more detailed information about the phase distribution of the sintered samples.

Raman spectra, which are shown in Figure 1b, present the characteristic vibrational fingerprint of HAp (corresponding to the $PO_4{}^{3-}$ internal modes), enabling to discard both the pluronic hydrogel residues (since no peaks related to pluronic identity were detected) and the presence of other calcium phosphate salts. Thus, the latter would be unequivocally identified by a characteristic splitting or a shifting of the main peak at $v_1$ = 962 cm$^{-1}$, which corresponds to the P-O symmetric stretching mode. Moreover, such peaks become sharper and better defined after sintering, indicating that crystallinity increases. Additionally, the peak areas at 3574 cm$^{-1}$, which are associated to the O-H stretching mode and normalized by the area of the $v_1$ ($A_{3574}$), were evaluated to control the proper generation of OH$^-$ vacancies during the dehydration process (Figure 1c). The obtained results clearly show that the presence of pluronic hydrogel does not affect the final amount of generated vacancies, as long as $A_{3574}$ = 0.160 $\pm$ 0.003 and 0.167 $\pm$ 0.002 for s-HAp and s/50-HAp are almost 33% times lower than the value obtained for as prepared HAp. Therefore, it can be concluded that the sintering process successfully suppresses the hydrogel from the HAp scaffold without affecting the final composition of the mineral.

## 8. Influence of pluronic hydrogel on the final HAp scaffolds

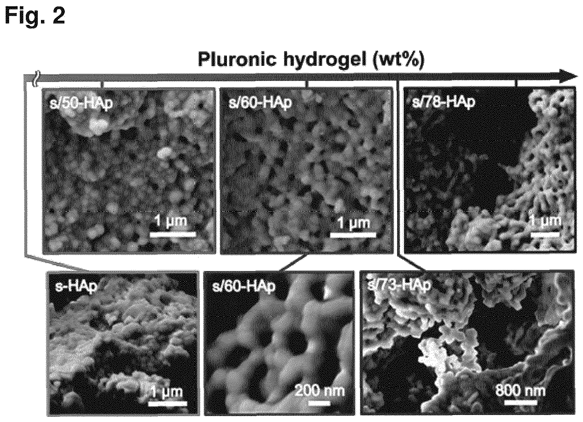

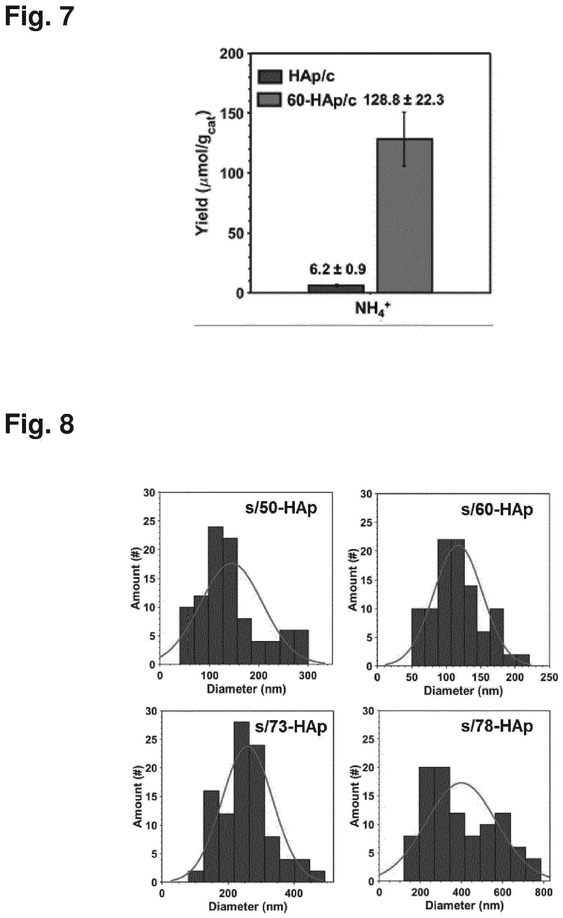

Figure 2 compares SEM micrographs of s-HAp and s/x-HAp, which were obtained using different pluronic hydrogel mass percentages (i.e. 50%, 60%, 73% and 78%). The generation of spherical nano- and submicrometric pores (from 120 $\pm$ 73 to 400 $\pm$ 122 nm) was clearly observed in all s/x-HAp samples. Nano- and submicrometric pores consistently presented normal distributions for all samples (Figure 8), even though a new family of micrometric cavities appeared when the mass percentage of pluronic hydrogel increased from 50 % to 73% wt. and 78% wt (Figure 2). This phenomenon has been attributed to the effect of residual hydrogel that was not properly dispersed among the HAp powder. Due to the fact that their sizes presented a poor dependence on the mass percentage of pluronic hydrogel (6.2 $\pm$ 2.0 $\mu$m and 5.4 $\pm$ 2.8 $\mu$m for s/73-HAp and s/78-HAp, respectively), both types of pores coexisted independently.

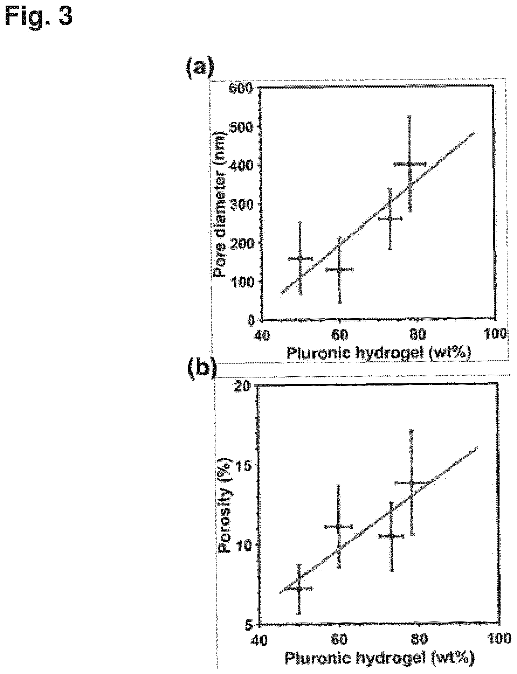

The porosity of samples associated to nano- and submicrometric pores (i.e. discarding micrometric cavities), which was obtained considering the percentage of void space observed in SEM images, ranged from 6% to 14%. As is illustrated in Figure 2 for s/60-HAp, high magnification images allow discerning how pluronic hydrogel droplets were the precursors of the resulting pores, favoring the location of HAp sintered grains in their surroundings. Figure 3 shows that the pore size and the porosity of s/x-HAp depend on the size and number of hydrogel droplets, evidencing that the former can be controlled through the mass percentage of hydrogel added to the initial mixture.

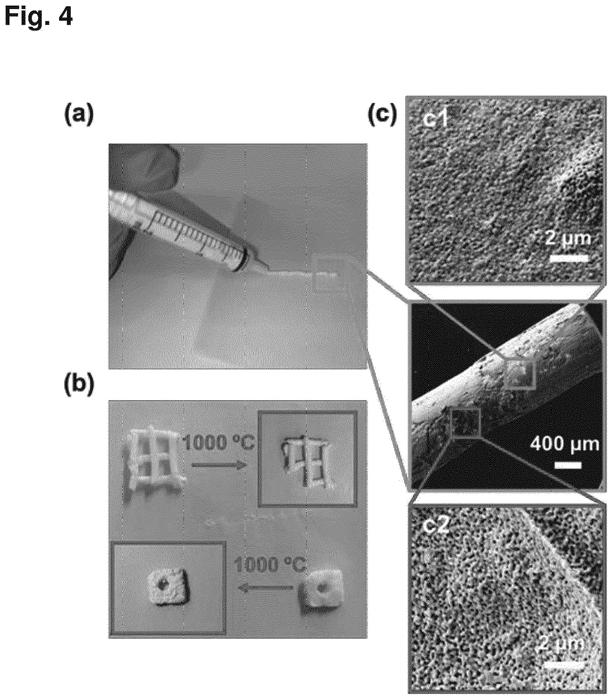

3D printed HAp scaffolds were achieved by extruding the pluronic hydrogel-HAp mixture, which exhibited good rheological conditions, as the hydrogel bound the HAp powder, when it was maintained at low temperatures (< 4 °C). In order to obtain the optimum paste properties for 3D printing, different HAp inks were preliminary examined by varying the mass percentage of the hydrogel. Finally, a pluronic hydrogel mass percentage of ~ 60% was considered for further studies due to its optimal printable properties, good mechanical stability and control of pore size and porosity (see below). Figure 4a-b shows that such HAp ink was viscous enough to be introduced into a syringe but sufficiently dry to maintain the desired shape even at cold temperatures, when pluronic hydrogel was still liquid. Moreover, sintered samples maintained the desired shape without showing observable cracks or shrinkage, and with good structural stability with fair bonding between filaments (Figure 4b).

SEM images acquired from extruded filaments revealed that the porosity at the exposed surface decreased to 4 % (Figure 4c1), which has been attributed to the friction with the walls and suggesting that such behavior could be a drawback. However, focusing on a broken region of the rod where the inner part (or the cavity) can be observed, the porosity recovers the standard value of 12 % (Figure 4c2), which is in agreement with that was found for s/60-HAp samples (Figure 3b). Even though the surface porosity could be enhanced by using coated syringes and/or more viscous HAp inks, together with a precise control of the temperature, the inventors' results indicate that the utilization of a syringe presents significant handling advantages.

Further, water absorption capability studies comparing s-HAp and s/60-HAp scaffolds were conducted. To enhance their porosity, s/60-HAp scaffolds were shaped as cubes (Figure 4b), whereas s-HAp consisted of disks made of compressed HAp powder. The below table 2 shows that water flow absorption was almost four times greater for s/60-HAp than for s-HAp, which was mainly attributed to the pores of the former.

**Table 2.** Water absorption capability of different samples before (s-HAp and s/60-HAp) and after (HAp/c and 60-HAp/c) catalytic activation.

| | Samples s-HAp | s/60-Hap | HAp/c | 60-HAp/c |
|---|---|---|---|---|
| **Water flow absorption [$\mu$L/s]** | 0.93 $\pm$ 0.14 | 3.57 $\pm$ 0.17 | 1.73 $\pm$ 0.39 | 2.60 $\pm$ 0.04 |

The same study was performed after catalytic activation of s-HAp and s/60-HAp, which produced the HAp/c and 60-HAp/c catalysts by applying the TSP process. The same experimental conditions (Methods section) were applied to both samples. Although the polarization process is known to affect the hydrophilicity of the samples due to a surface charge induction effect (Rivas, M.; del Valle, L. J.; Armelin, E.; Bertran, O.; Turon, P.; Puiggalí, J.; Alemán, C. Hydroxyapatite with Permanent Electrical Polarization: Preparation, Characterization, and Response against Inorganic Adsorbates. ChemPhysChem 2018, 19, 1746-1755), the enhancement of water absorption capability was only observed for HAp/c, which has been attributed to the non-negligible effect of increased roughness. Despite of this, 60-HAp/c still presented much better absorption capability than HAp/c (i.e. 1.5 times greater), which is expected to promote the final catalytic activity of the carbon and nitrogen fixation reactions.

9. Catalytic activity of 60-HAp/c

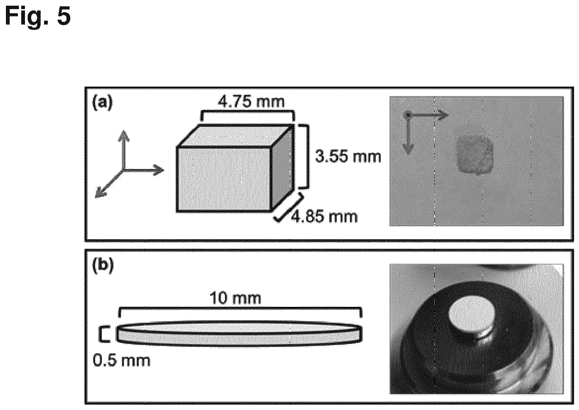

In order to elucidate the effect of porosity on the catalytic activity of the HAp-based catalysts, 60-HAp/c cubes were prepared and compared with HAp/c disks (Figure 5). For this purpose, s/60-HAp cubes and s-HAp disks were polarized using identical experimental conditions. The porosity was calculated in previous sections considering SEM images of grains and comparing different pluronic hydrogel loadings. However, the utilization of specific geometrical parameters, as defined in Figure 5, instead of grains allows an alternative expression of relative porosity ($\Pi_{rel}$) measured by gravimetry, which appears to be more appropriate for comparing both samples from a catalytic point of view. Hence:

$$\Pi_{rel} = 1 - \rho_{60\text{-HAp/c}} / \rho_{HAp/c} \qquad (1)$$

where $\rho_{60\text{-HAp/c}}$ and $\rho_{PHAp/c}$ are the densities of 60-HAp/c cubes and HAp/c disks, respectively, which were obtained using the parameters described in the below table 3. The resulting value, $\Pi_{rel} = 0.7 \pm 0.03$, indicates that the difference between the porosities of the materials is higher than the one obtained by SEM inspection. Indeed, $\Pi_{rel}$ suggests greater porosity with good pore interconnectivity in the bulk, and confirms the fact that external handling of the HAp inks results in a reduction of superficial porosity due to abrasion.

**Table 3**. Macroscopic parameters of the 60-HAp/c and HAp/c catalysts (Figure 5). The exposed area only considers the faces of the geometry that are exposed in the reaction.

|  | Exposed Area [mm$^2$] | Volume [mm$^3$] | Weight [mg] |
| --- | --- | --- | --- |
| 60-HAp/c (cube) | 74.3 | 81.8 | 132 |
| c-HAp (disk) | 94.3 | 39.3 | 200 |

The catalytic performance of the 60-HAp/c and HAp/c was compared for two different reactions based on nitrogen and carbon fixation: *i*) the production of ethanol using mixtures of $CO_2$ and $CH_4$ (Sans, J.; Revilla-López, G.; Sanz, V.; Puiggalí, J.; Turon, P.; Alemán, C. Permanently Polarized Hydroxyapatite for Selective Electrothermal Catalytic Conversion of Carbon Dioxide into Ethanol. Chem. Commun. 2021, 57, 5163-5166; and *ii*) the conversion of $N_2$ to ammonia. As it is shown below, the conversion of $CO_2$ and $CH_4$ to ethanol and of $N_2$ to ammonia improves by around 3000% and 2000%, respectively, in comparison to HAp/c, as discussed below.

10. Carbon and dinitrogen fixation reactions

Catalysts were introduced in an inert reactor and tested for two reported electrothermal catalytic reactions: 1) the synthesis of ethanol from $CO_2$ and $CH_4$; and 2) the conversion of $N_2$ to ammonia. Quantification of the reaction yields was performed using $^1$H-NMR spectroscopy. Specific details are provided in the following.

The reactor comprised an inert reaction chamber coated with a perfluorinated polymer (120 mL), in which both the catalyst and water (1 mL) were incorporated. The reactor was equipped with an inlet valve for the entrance of $N_2$, $CH_4$, $CO_2$ and an outlet valve to recover the gaseous reaction products. A UV lamp (GPH265T5L/4, 253.7 nm) was also placed in the middle of the reactor to irradiate the catalyst directly, the lamp being protected by a UV transparent quartz tube. All surfaces were coated with a thin film of a perfluorinated polymer in order to avoid any contact between the reaction medium and the reactor surfaces, in this way discarding other catalyst effects.

The reaction products were analyzed by $^1$H NMR spectroscopy. All $^1$H NMR spectra were acquired with a Bruker Avance III-400 spectrometer operating at 400.1 MHz. The chemical shift was calibrated using tetramethylsilane as internal standard. Sixty-four scans were recorded in all cases. In order to remove the products formed on the catalysts from reactions involving $CO_2$ and $CH_4$, samples were dissolved in deuterated water containing 100 mM of HCl and 50 mM of NaCl with the final addition of deuterated water.

In the case of the dinitrogen fixation reaction to produce ammonia, the catalyst (10 mg) was dissolved in 15 mL of water with pH adjusted to $2.1 \pm 0.2$ using 7.6 mM $H_2SO_4$, to promote the conversion of ammonia in $NH_4^+$, and applying 4 cycles that involved sonication (5 min) and stirring (1 min) steps. Then, for the $^1$H NMR sample preparation, 500 $\mu$L of the reacted catalyst solution were mixed with 100 $\mu$L of DMSO-$d_6$ instead of solvents with labile deuterons (*i.e.* $D_2O$) to avoid the formation of ammonium deuterated analogues, not desired for quantitative analysis. The same treatment was applied to the water supernatant.

*10.1 Production of ethanol using* $CO_2$ *and* $CH_4$

The performance of 60-HAp/c and HAp/c catalysts for the production of ethanol have been compared. The reaction was performed using the same reactor chamber under a $CO_2$ and $CH_4$ atmosphere (3 bar each), at 140 °C but without the presence of UV light for 48 h. Initially, $CO_2$ was used to purge the reactor. The catalyst, HAp/c disks or 60-HAp/c cubes without any coating, and 1 mL of de-ionized liquid water were incorporated into the reaction chamber (reactions were performed separately for each catalyst). Additionally, the effect of the initial water content on the reaction yield was investigated for one of the 60-HAp/c catalysts.

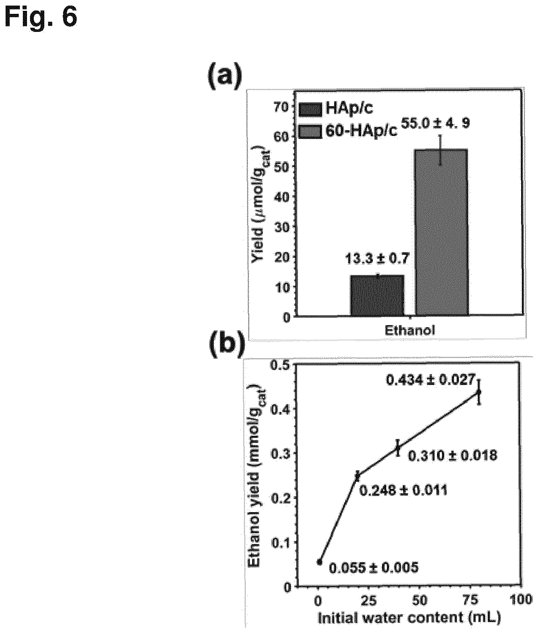

The ethanol yields obtained for both 60-HAp/c and HAp/c are compared in Figure 6a. The ethanol production is around four times higher for the former than for the latter ($55.0 \pm 4.9$ and $13.3 \pm 0.7$ $\mu$mol per gram of catalyst, respectively), which represents an outstanding increment of the catalytic performance (414 %).

Previous studies evidenced that water is necessary as proton source, whereas the excess of water content hinders the gas fixation onto the HAp/c surface, diminishing the final yields of the reaction (Sans, J.; Revilla-López, G.; Sanz, V.; Puiggalí, J.; Turon, P.; Alemán, C. Permanently Polarized Hydroxyapatite for Selective Electrothermal Catalytic Conversion of Carbon Dioxide into Ethanol. Chem. Commun. 2021, 57, 5163-5166). In this work the examiner have examined if the water content is still a limiting factor for 60-HAp/c, which is characterized by both the generation of pores and the high water absorption in comparison to HAp/c. Accordingly, a series of reactions varying the initial water content from 1 to 80 mL, while the $CO_2$ and $CH_4$ pressures (3 bar each), the temperature (140 °C) and the reaction time (48 h) were kept.

The yield of ethanol, which is plotted in Figure 6b, increased progressively with the increase of initial water introduced in the reactor, even when the initial water content is 80 times greater than that used in the standard reaction for HAp/c (1 mL). This result describes the huge catalytic potential of 60-HAp/c that is able to produce up to 434 $\pm$ 27 $\mu$mol per gram of catalyst when the water content is 80 mL, reaching for the first time a production comparable to the one reported for cutting edge Cu-based catalysts (Ma, W.; Xie, S.; Liu, T.; Fan, Q.; Ye, J.; Sun, F.; Jiang, Z.; Zhang, Q.; Cheng, J.; Wang, Y. Electrocatalytic Reduction of CO2 to Ethylene and Ethanol through Hydrogen-Assisted C-C Coupling over Fluorine-Modified Copper. Nat. Catal. 2020, 3, 478-487). Moreover, it is worth noting that Cu-based catalysts require the application of electric potentials, whereas no potential is necessary for 60-HAp/c performance. On the other hand, the ethanol or any other product in the remaining water inside the reactor was found to be null, highlighting the absorption capability of the catalyst and stressing out the scalability of it. Overall, it can be concluded that porosity boosts the catalytical performance of HAp/c for the production of ethanol, obtaining for the first time yields high enough for industrial scalability and feasibility.

*10.2 Conversion of $N_2$ to ammonia*

The production of ammonia using 60-HAp/c and HAp/c catalysts was performed at 120 °C and under UV illumination. In order to eliminate the initial air content, the reaction chamber was firstly purged with $N_2$ and, subsequently, filled with $N_2$ until a pressure of 6 bar was reached. A volume of 20 mL of de-ionized water was introduced in the reactor and put in contact with the nonirradiated side of the catalyst. It is worth noting that in this reaction water acted not only as the proton source for the ammonia formation (from water splitting) but also as a medium to facilitate the recovery of the formed product. The product generated on the surface of the catalysts as well as the product collected in liquid water after 24 h of reaction was identified as $NH_4^+$ adapting a procedure based on $^1$H NMR spectroscopy (Hodgetts, R. Y.; Kiryutin, A. S.; Nichols, P.; Du, H.-L.; Bakker, J. M.; Macfarlane, D. R.; Simonov, A. N. Refining Universal Procedures for Ammonium Quantification via Rapid 1H NMR Analysis for Dinitrogen Reduction Studies. ACS Energy Lett. 2020, 5, 736-741).

Results, which are depicted in Figure 7, showed that the total amount of $NH_4^+$ formed in presence of the HAp/c was of 6.2 $\pm$ 0.9 $\mu$mol per gram of catalyst. Although around 25% of the formed $NH_4^+$ (*i.e.* 1.7 $\pm$ 0.3 $\mu$mol per gram of catalyst) remained adsorbed onto the catalyst surface, the main part of the reaction product was transferred from the catalyst to the water medium (i.e. 4.5 $\pm$ 0.6 $\mu$mol per gram of catalyst). The influence of the porosity and water absorption capacity on the yield of $NH_4^+$ was dramatic, the amount of product formed in presence of 60-HAp/c increasing to 128.8 $\pm$ 22.3 $\mu$mol per gram of catalyst. Moreover, the total of the yield, which represented an increment of more than 2000% with respect to HAp/c, was collected on the remaining liquid water. This feature, which is fully consistent with the results obtained in the previous reaction, corroborates that 60-HAp/c enhances the synthesis of the reaction products.

Porous HAp scaffolds with controlled architecture have been successfully created by mixing HAp powder with pluronic hydrogel. This composition of this mixture allows to regulate the properties of the resulting ink to fulfill 3D-printing requirements, proper mechanical stability being achieved by sintering at high temperatures. Sintered HAp scaffolds exhibit high purity and crystallinity, reflecting a correct dehydration process. Therefore, the addition of pluronic hydrogel for enabling the printable inks, and their posterior generation of pores, does not affect the structure required for preparing polarized HAp-based catalysts.

The catalytic activity of the porous samples has been evaluated using different carbon and/or dinitrogen fixation reactions. The 60-HAp/c catalyst showed an outstanding increment of the yields of the reaction. This has been mainly attributed to the enhanced water absorption capability and higher exposed surface. More specifically, the presence of micro-cavities inside the catalyst promotes the heterogeneous catalytic processes used for the production of ethanol and ammonia. Overall, its catalytic activity and huge scalability potential, postulates 60-HAp/c as a solid, cheaper and environmentally friendly alternative to other conventional catalysts.

**Claims**

1. Porous permanently polarized hydroxyapatite, wherein the porous permanently polarized hydroxyapatite has a mean pore diameter from 10 nm to 500.000 nm.

2. The porous permanently polarized hydroxyapatite according to claim 1, **characterized in that** the mean pore diameter is from 10 nm to 10.000 nm, in particular 50 nm to 1.000 nm, preferably 60 nm to 600 nm, more preferably 100 nm to 350 nm.

3. The porous permanently polarized hydroxyapatite according to claim 1 or 2, **characterized in that** the porous permanently polarized hydroxyapatite has nanometric pores and submicrometric pores or nanometric pores, submicrometric pores and micrometric pores.

4. The porous permanently polarized hydroxyapatite according claim 3, **characterized in that**

   - the nanometric pores have a mean pore diameter from 1 nm to 100 nm, in particular 10 nm to 90 nm, preferably 50 nm to 80 nm,
   and/or
   - the submicrometric pores have a mean pore diameter from 101 nm to 999 nm, in particular 120 nm to 900 nm, preferably 150 nm to 200 nm,
   and/or
   - the micrometric pores have a mean pore diameter from 1 $\mu$m to 500 $\mu$m, in particular 2 $\mu$m to 100 $\mu$m, preferably 5 $\mu$m to 10 $\mu$m.

5. The porous permanently polarized hydroxyapatite according to any of the preceding claims, **characterized in that** the porous permanently polarized hydroxyapatite has

   - a wide angle X-ray scattering (WAXS) pattern as shown on Fig. 1(a)
   and/or
   - a Raman spectrum as shown on Fig. 1(b)
   and/or
   - a $^{31}$P-NMR spectrum as shown on Fig. 1(d).

6. A process for obtaining a porous permanently polarized hydroxyapatite according to any of the preceding clams comprising the steps of:

   (a) providing a mixture comprising or consisting of hydroxyapatite powder and a pore-forming material,
   (b) shaping the mixture provided in step (a) to obtain a shaped body comprising or consisting of the hydroxyapatite powder and the pore-forming material,
   (c) sintering the shaped body obtained in step (b) to remove the pore-forming material and to obtain a shaped body comprising or consisting of porous hydroxyapatite,
   (d) applying a constant or variable DC voltage, in particular between 250 V and 2550 V, or an equivalent electric field, in particular between 1.49 kV/cm and 15 kV/cm,

      or
      applying an electrostatic discharge, in particular between 2500 V and 1500000 V, or an equivalent electrical field, in particular between 148.9 kV/cm and 8928 kV/cm, to the shaped body obtained in step (c) to obtain a shaped body comprising or consisting of porous permanently polarized hydroxyapatite
      and

   (e) cooling the shaped body obtained in step (d) maintaining the DC voltage or the equivalent electric field applied in step (d)
   or

      cooling the shaped body obtained in step (d) maintaining the electrostatic discharge or the equivalent electric field applied in step (d)
      or
      cooling the shaped body obtained in step (d) without maintaining the DC voltage or the equivalent electric field applied in step (d)
      or
      cooling the shaped body obtained in step (d) without maintaining the electrostatic discharge or the equivalent electric field applied in step (d).

7. The process according to claim 6, **characterized in that** the pore-forming material comprises a polymer, in particular selected from the group consisting of poloxamer, polyolefines, polyesters, polyamides, polyimides, polyvinyl alcohols, polyurethanes, polycarbonates, polyalkylterephthalates, polyarylterephthalates, polyaryletherketones, polyhydroxyalkanoates, proteins such as extracellular proteins and/or globular proteins and/or enzymes and/or antibodies and/or blood clotting factors, polysaccharides and mixtures of at least two of the afore-said polymers.

8. The process according to any of the claims 9 to 11, **characterized in that** the polymer is a poloxamer.

9. The process according to claim 7 or 8, **characterized in that** the poloxamer comprises a a polyoxypropylene core having a molecular mass of 1 g/mol to 500.000 g/mol, in particular 1.000 g/mol to 30.000 g/mol, preferably 10.000 g/mol to 20.000 g/mol.

10. The process according to any of the claims 7 to 9, **characterized in that** the poloxamer has a polyoxyethylene content from 1 % by weight to 90 % by weight, in particular 10 % by weight to 80 % by weight, preferably 35 % by weight to 65 % by weight.

11. The process according to any of the claims 7 to 10, **characterized in that** the polymer has a proportion 1 % by weight to 90 % by weight, preferably 20 % by weight to 80 % by weight, more preferably 35 % by weight to 65 % by weight, based on the total weight of the pore-forming material.

12. The process according to any of the claims 7 to 11, **characterized in that** the pore-forming material is in the form of a hydrogel.

13. A porous permanently polarized hydroxyapatite obtained or obtainable by a process according to any of the claims 6 to 12.

14. Composition or material, in particular in the form of a catalyst, comprising a porous permanently polarized hydroxyapatite according to any of the claims 1 to 5 or 13.

15. Use of a porous permanently polarized hydroxyapatite according to any of the claims 1 to 5 or 13 or of a composition or material according to claim 14 as a catalyst, in particular in a reaction for the synthesis of organic molecules, preferably ethanol, or inorganic molecules, preferably ammonia.

16. Composition, in particular a pasty or ink composition, for preparing a porous permanently polarized hydroxyapatite according to any of the claims 1 to 5 or 13, comprising hydroxyapatite powder and a pore-forming material.

# Fig. 1

# Fig. 1 (d)

Fig. 2

Fig. 3

(a)

(b)

## Fig. 4

(a)

(b)

(c)

# Fig. 5

Fig. 6

## Fig. 7

## Fig. 8

Europäisches
Patentamt

European
Patent Office

Office européen
des brevets

# EUROPEAN SEARCH REPORT

**Application Number**

EP 22 38 2034

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 278 818 A1 (B BRAUN SURGICAL SA [ES]; UNIV POLITÈCNICA DE CATALUNYA [ES]) 7 February 2018 (2018-02-07) | 1-5, 13-15 | INV. B01J27/18 C01B25/32 |
| A | * claims 1-15 * <br> * paragraphs [0034] - [0038] * <br> * figures 4,11,18c * | 6-12,16 | C07C29/00 C07C31/02 C10L1/02 |
| X | GITTINGS J. P. ET AL: "Influence of Porosity on Polarisation and Electrical Properties of Hydroxyapatite Based Ceramics", FERROELECTRICS, vol. 390, no. 1, 28 October 2009 (2009-10-28), pages 168-176, XP055934949, US ISSN: 0015-0193, DOI: 10.1080/00150190903001011 * the whole document * | 1-16 | |
| A | WO 2021/219644 A1 (B BRAUN SURGICAL SA [ES]; UNIV CATALUNYA POLITECNICA [ES]) 4 November 2021 (2021-11-04) * claims 1-16 * | 1-16 | **TECHNICAL FIELDS SEARCHED (IPC)** <br><br> B01J C10G C01B C07C C10L |

The present search report has been drawn up for all claims

| Place of search | Date of completion of the search | Examiner |
|---|---|---|
| The Hague | 23 June 2022 | Gerwann, Jochen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## EP 4 215 271 A1

This annex lists the patent family members relating to the patent documents cited in the above-mentioned European search report.
The members are as contained in the European Patent Office EDP file on
The European Patent Office is in no way liable for these particulars which are merely given for the purpose of information.

23-06-2022

| Patent document cited in search report | | Publication date | Patent family member(s) | | Publication date |
|---|---|---|---|---|---|
| EP 3278818 | A1 | 07-02-2018 | EP | 3278818 A1 | 07-02-2018 |
| | | | ES | 2902489 T3 | 28-03-2022 |
| WO 2021219644 | A1 | 04-11-2021 | EP | 3953021 A1 | 16-02-2022 |
| | | | WO | 2021219644 A1 | 04-11-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **GAITZSCH, J. ; HUANG, X. ; VOIT, B.** Engineering Functional Polymer Capsules toward Smart Nanoreactors. *Chem. Rev.,* 2016, vol. 116, 1053-1093
- **RENGGLI, K. ; BAUMANN, P. ; LANGOWSKA, K. ; ONACA, O. ; BRUNS, N. ; MEIER, W.** Selective and Responsive Nanoreactors. *Adv. Funct. Mater.,* 2011, vol. 21, 1241-1259
- **VRIEZEMA, D. M. ; ARAGONÈS, M. C. ; ELEMANS, J. A. A. W. ; CORNELISSEN, J. J. L. M. ; ROWAN, A. E. ; NOLTE, R. J. M.** Self-Assembled Nanoreactors. *Chem. Rev.,* 2005, vol. 105, 1445-1489
- **REVILLA-LÓPEZ, G. ; SANS, J. ; CASANOVAS, J. ; BERTRAN, O. ; PUIGGALÍ, J. ; TURON, P. ; ALEMÁN, C.** Analysis of Nitrogen Fixation by a Catalyst Capable of Transforming N2, CO2 and CH4 into Amino Acids under Mild Reactions Conditions. *Appl. Catal. A Gen.,* 2020, vol. 596, 117526
- **SANS, J. ; REVILLA-LÓPEZ, G. ; SANZ, V. ; PUIGGALÍ, J. ; TURON, P. ; ALEMÁN, C.** Permanently Polarized Hydroxyapatite for Selective Electrothermal Catalytic Conversion of Carbon Dioxide into Ethanol. *Chem. Commun.,* 2021, vol. 57, 5163-5166
- **SANS, J. ; SANZ, V. ; L. J. DEL VALLE ; PUIGGALÍ, J. ; TURON, P. ; ALEMAN, C.** Optimization of Permanently Polarized Hydroxyapatite Catalyst. Implications for the Electrophotosynthesis of Amino Acids by Nitrogen and Carbon Fixation. *J. Catal.,* 2021, vol. 397, 98-107

- **SANS, J. ; ARNAU, M. ; TURON, P. ; ALEMAN, C.** Article OH-. Regulating the Superficial Vacancies and OH- Orientations on Polarized Hydroxyapatite Electrocatalysts. *Adv. Mater. Interfaces,* 2021
- **RIVAS, M. ; DELVALLE, L. J. ; ARMELIN, E. ; BERTRAN, O. ; TURON, P. ; PUIGGALÍ, J. ; ALEMÁN, C.** Hydroxyapatite with Permanent Electrical Polarization: Preparation, Characterization, and Response against Inorganic Adsorbates. *ChemPhysChem,* 2018, vol. 19, 1746-1755
- **RIVAS, M. ; DEL VALLE, L. J. ; ARMELIN, E. ; BERTRAN, O. ; TURON, P. ; PUIGGALÍ, J. ; ALEMÁN, C.** Hydroxyapatite with Permanent Electrical Polarization: Preparation, Characterization, and Response against Inorganic Adsorbates. *ChemPhysChem,* 2018, vol. 19, 1746-1755
- **SANS, J. ; REVILLA-LÓPEZ, G. ; SANZ, V. ; PUIGGALÍ, J. ; TURON, P. ; ALEMÁN, C.** Permanently Polarized Hydroxyapatite for Selective Electrothermal Catalytic Conversion of Carbon Dioxide into Ethanol. *Chem. Commun.,* 2021, vol. 57, 5163-5166
- **MA, W. ; XIE, S. ; LIU, T. ; FAN, Q. ; YE, J. ; SUN, F. ; JIANG, Z. ; ZHANG, Q. ; CHENG, J. ; WANG, Y.** Electrocatalytic Reduction of CO2 to Ethylene and Ethanol through Hydrogen-Assisted C-C Coupling over Fluorine-Modified Copper. *Nat. Catal.,* 2020, vol. 3, 478-487
- **HODGETTS, R. Y. ; KIRYUTIN, A. S. ; NICHOLS, P. ; DU, H.-L. ; BAKKER, J. M. ; MACFARLANE, D. R. ; SIMONOV, A. N.** Refining Universal Procedures for Ammonium Quantification via Rapid H NMR Analysis for Dinitrogen Reduction Studies. *ACS Energy Lett.,* 2020, vol. 5, 736-741